Europäisches Patentamt

(19) European Patent Office

Office européen des brevets

(11) Publication number: **0 321 268**
**A2**

(12) # EUROPEAN PATENT APPLICATION

(21) Application number: **88311919.0**

(22) Date of filing: **16.12.88**

(51) Int. Cl.⁴: **C 12 N 15/00**
**C 12 N 9/10, C 12 N 9/22,**
**C 12 N 1/20**

(30) Priority: **17.12.87 US 133941**

(43) Date of publication of application:
**21.06.89 Bulletin 89/25**

(84) Designated Contracting States:
**AT BE CH DE ES FR GB GR IT LI LU NL SE**

(71) Applicant: **NEW ENGLAND BIOLABS, INC.**
**32 Tozer Road**
**Beverly Massachusetts 01915 (US)**

(72) Inventor: **Camp, Russell**
**35, Village Lane**
**Hamilton Massachusetts 01936 (US)**

**Wilson, Geoffrey**
**10, Long Hill**
**Boxford Massachusetts 01920 (US)**

(74) Representative: **Geering, Keith Edwin et al**
**REDDIE & GROSE 16 Theobalds Road**
**London WC1X 8PL (GB)**

(54) Method for producing the Banl restriction endonuclease and methylase.

(57) The present invention is directed to a method for cloning and producing the Banl restriction endonuclease by 1) introducing the restriction endonuclease gene from *B.aneurinolyticus* IAM 1077 into a host whereby the restriction gene is expressed; 2) fermenting the host which contains the vector encoding and expressing the Banl restriction endonuclease, and 3) purifying the Banl restriction endonuclease from the fermented host which contains the vector encoding and expressing the Banl restriction endonuclease activity.

Figure 1

Description

# METHOD FOR PRODUCING THE BanI RESTRICTION ENDONUCLEASE AND METHYLASE

## BACKGROUND OF THE INVENTION

The present invention relates to clones for the BanI restriction endonuclease and modification methylase, and to the production of these enzymes from the clones.

Restriction endonucleases are a class of enzymes that occur naturally in bacteria. When they are purified away from other contaminating bacterial components, restriction endonucleases can be used in the laboratory to break DNA molecules into precise fragments. This property enables DNA molecules to be uniquely identified and to be fractionated into their constituent genes. Restriction endonucleases have proved to be indispensable tools in modern genetic research. They are the biochemical 'scissors' by means of which genetic engineering and analysis is performed.

Restriction endonucleases act by recognizing and binding to particular sequences of nucleotides (the 'recognition sequence') along the DNA molecule. Once bound, they cleave the molecule within, or to one side of, the sequence. Different restriction endonucleases have affinity for different recognition sequences. Over one hundred different restriction endonucleases have been identified among many hundreds of bacterial species that have been examined to date.

Bacteria usually possess only a small number restriction endonucleases per species. The endonucleases are named according to the bacteria from which they are derived. Thus, the species *Haemophilus aegyptius*, for example synthesizes 3 different restriction endonucleases, named HaeI, HaeII and HaeIII. These enzymes recognize and cleave the sequences (AT)GGCC(AT), PuGCGCPy and GGCC respectively. *Escherichia coli* RY13, on the other hand, synthesizes only one enzyme, EcoRI, which recognizes the sequence GAATTC.

While not wishing to be bound by theory, it is thought that in nature, restriction endonucleases play a protective role in the welfare of the bacterial cell. They enable bacteria to resist infection by foreign DNA molecules like viruses and plasmids that would otherwise destroy or parasitize them. They impart resistance by binding to infecting DNA molecule and cleaving them each time that the recognition sequence occurs. The disintegration that results inactivates many of the infecting genes and renders the DNA susceptible to further degradation by exonucleases.

A second component of bacterial protective systems are the modification methylases. These enzymes are complementary to restriction endonucleases and they provide the means by which bacteria are able to protect their own DNA and distinguish it from foreign, infecting DNA. Modification methylases recognize and bind to the same nucleotide recognition sequence as the corresponding restriction endonuclease, but instead of breaking the DNA, they chemically modify one or other of the nucleotides within the sequence by the addition of a methyl group. Following methylation, the recognition sequence is no longer bound or cleaved by the restriction endonuclease. The DNA of a bacterial cell is always fully modified, by virtue of the activity of its modification methylase and it is therefore completely insensitive to the presence of the endogenous restriction endonuclease. It is only unmodified, and therefore identifiably foreign, DNA that is sensitive to restriction endonuclease recognition and attack.

With the advent of genetic engineering technology, it is now possible to clone genes and to produce the proteins and enzymes that they encode in greater quantities than are obtainable by conventional purification techniques. The key to isolating clones of restriction endonuclease genes is to develop a simple and reliable method to identify such clones within complex 'libraries', i.e. populations of clones derived by 'shotgun' procedures, when they occur at frequencies as low as $10^{-3}$ to $10^{-4}$. Preferably, the method should be selective, such that the unwanted, majority, of clones are destroyed while the desirable, rare, clones survive.

Type II restriction-modification systems are being cloned with increasing frequency. The first cloned systems used bacteriophage infection as a means of identifying or selecting restriction endonuclease clones (HhaII: Mann *et al,, Gene* 3: 97-112, (1978); EcoRII: Kosykh *et al., Molec. gen. Genet* 178: 717-719, (1980); PstI: Walder *et al., Proc. Nat. Acad. Sci. USA* 78 1503-1507, (1981)). Since the presence of restriction-modification systems in bacteria enables them to resist infection by bacteriophages, cells that carry cloned restriction-modification genes can, in principle, be selectively isolated as survivors from libraries that have been exposed to phage. This method has been found, however, to have only limited value. Specifically, it has been found that cloned restriction-modification genes do not always manifest sufficient phage resistance to confer selective survival.

Another cloning approach involves transferring systems initially characterized as plasmid-borne into *E.coli* cloning plasmids (EcoRV: Bougueleret *et al., Nucleic Acids Res.* 12:3659-3676, (1984); PaeR7: Gingeras and Brooks, *Proc. Natl. Acad. Sci. USA* 80:402-406, (1983); Theriault and Roy, *Gene* 19:355-359, (1982); PvuII: Blumenthal *et al., J.Bacteriol.* 164:501-509, (1985)).

A third approach, and one that is being used to clone a growing number of systems, involves selecting for an active methylase gene referring to our Patent application No. 707079 and (BsuRI: Kiss *et al., Nucleic Acids Res.* 13:6403-6421, (1985)). Since restriction and modification genes tend to be closely linked, clones containing both genes can often be isolated by selecting for just the one gene. Selection for methylation activity does not always yield a complete restriction-modification system however, but instead sometimes yields only the methylase gene (BspRI: Szomolanyi et al., Gene 10:219-225, (1980); BcnI: Janulaitis et al, Gene 20:197-204 (1982); BsuRI: Kiss and Baldauf, Gene 21:111-119, (1983); and MspI: Walder et al., J. Biol. Chem. 258:1235-1241, (1983)).

A potential obstacle to cloning restriction-modification genes lies in trying to introduce the endonuclease gene into a host not already protected by modification. If the methylase gene and endonuclease gene are introduced together as a single clone, the methylase must protectively modify the host DNA before the endonuclease has the opportunity to cleave it. On occasion, therefore, it might only be possible to clone the genes sequentially, methylase first then endonuclease. Another obstacle to cloning restriction-modification systems lies in the discovery that some strains of E.coli react adversely to cytosine modification; they possess systems that destroy DNA containing methylated cytosine (Raleigh and Wilson, Proc. Natl. Acad. Sci., USA 83:9070-9074, (1986)). Cytosine-specific methylase genes cannot be cloned easily into these strains, either on their own, or together with their corresponding endonuclease genes. To avoid this problem it is necessary to use mutant strains of E.coli (MCrA⁻ and MCrB⁻) in which these systems are defective.

Because purified restriction endonucleases, and to a lesser extent, modification methylases, are useful tools for characterizing and rearranging DNA in the laboratory, there is a commercial incentive to obtain strains of bacteria through recombinant DNA techniques that synthesize these enzymes in abundance. Such strains would be useful because they would simplify the task of purification as well as providing the means for production in commercially useful amounts.

## SUMMARY OF THE INVENTION

In accordance with the present invention there is provided a clone containing the genes for the BanI restriction endonuclease and modification methylase derived from Bacillus aneurinolyticus (IAM 1077), as well as related methods for the production of the enzymes. More specifically, this invention relates to clones which express the restriction endonuclease BanI, an enzyme which recognizes the DNA sequence GGPyPuCC and cleaves between the two G's. See Sugisaki, H., Maekawa,Y., Kanazawa, S. and Takanami, M. Nucleic Acids Res. 10: 5747-5752, (1982), the disclosure of which is hereby incorporated by reference herein. BanI restriction endonuclease produced in accordance with the present invention is substantially pure and free of the contaminants normally found in BanI preparations made by conventional techniques, such as that disclosed by Sugisaki et al, supra.

The preferred method for cloning this enzyme comprises forming a library containing the DNA from B.aneurinolyticus (IAM 1077), isolating those clones which contain DNA coding for the BanI modification methylase and screening among these to identify those that also contain the BanI restriction endonuclease gene.

## BRIEF DESCRIPTION OF THE DRAWINGS

Figure 1 illustrates the scheme for cloning and producing the BanI restriction endonuclease.

Figure 2 is a restriction map of a 7.5 Kb HindIII fragment of B.aneurinolyticus DNA that encodes the BanI restriction endonuclease and modification methylase. The fragment was cloned into the HindIII site of pBR322 (ATCC 37017) to create pGW118RM 2-1, then it was transferred into the HindIII site of pUC19 (ATCC 37254) to create pRC118RM 102-6 and pRC118RM 102-18.

Figure 3 is a photograph of an agarose gel demonstrating BanI restriction endonuclease activity in cell extracts of E.coli RR1 (ATCC 31343) carrying pGW118RM 2-1, pRC118RM 102-6, and pRC118RM 102-18.

## DETAILED DESCRIPTION OF THE INVENTION

The present invention relates to clones of the BanI restriction and modification genes, as well to the restriction endonuclease BanI produced from such clones. The BanI genes are cloned by a method which takes advantage of the fact that certain clones which are selected on the basis of containing and expressing the BanI modification methylase gene also contain the BanI restriction gene. The DNA of such clones is resistant to digestion, in vitro, by the BanI restriction endonuclease. This resistance to digestion affords a means for selectively isolating clones encoding the BanI methylase and restriction endonuclease.

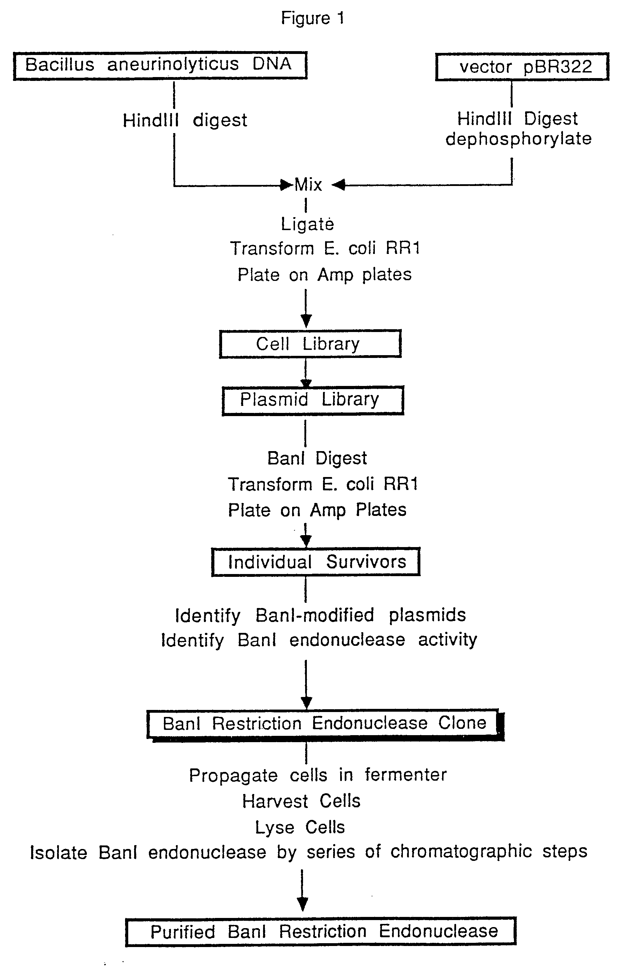

The method described herein by which the BanI restriction gene and methylase gene are preferably cloned and expressed are illustrated in Figure 1, and they include the following steps:

1. The DNA of Bacillus aneurinolyticus is purified. B.aneurinolyticus has been described by Sugisaki et al, supra. Samples of this bacterium are available from: The Institute of Applied Microbiology, University of Tokyo, Yayoi 1-1-1, Bunko-Ku, Tokyo, Japan 113, catalog # IAM 1077.

2. The DNA is digested with a restriction endonuclease such as HindIII.

3. The digested DNA is ligated to a cloning vector such as pBR322 (ATCC 37017), that contains one or more BanI sites. The ligated DNA is transformed into an appropriate host such as Escherichia coli strain RR1 (ATCC 31343).

4. The transformed mixture is plated onto media selective for transformed cells, such as the antibiotic ampicillin. After incubation, the transformed colonies are collected together into a single culture, the cell library.

5. The recombinant plasmids are purified in toto from the cell library to make the plasmid library.

6. The plasmid library is digested to completion with the BanI restriction endonuclease, prepared from *B.aneurinolyticus* by a method similar to that described in Sugisaki *et al, supra.* BanI digestion differentially destroys unmodified, non-methylase-containing, clones, increasing the relative frequency of BanI methylase clones.

7. The digested plasmid library is transformed back into an appropriate host such as *E.coli* RR1, and transformants are recovered by plating onto selective media. The colonies are picked and their DNA is analyzed for the presence of the BanI modification gene: the plasmids that they carry are purified and incubated with the BanI restriction endonuclease to determine whether they are resistant to digestion. Total cellular DNA (chromosomal and plasmid) is also purified and incubated with the BanI restriction endonuclease. The DNA of clones that carry the BanI modification gene should be fully modified, and both plasmid DNA and total DNA should be substantially resistant to digestion.

8. Clones carrying the BanI restriction endonuclease are identified by preparing cell extracts of the BanI methylase clones, identified in step 8, and assaying the extracts for BanI restriction endonuclease activity.

9. The quantity of BanI restriction endonuclease produced by the clones may be increased by elevating the gene dosage, through the use of high copy number vectors, and by elevating the transcription rate, through the use of highly active, exogenous promotors.

10. The BanI restriction endonuclease may be produced from clones carrying the BanI restriction and modification genes by propagation in a fermenter in a rich medium containing ampicillin. The cells are collected by centrifugation and disrupted by sonication to produce a crude cell extract containing the BanI restriction endonuclease activity.

11. The crude cell extract containing the BanI restriction endonuclease activity is purified by standard protein purification techniques such as affinity-chromatography and ion-exchange chromatography.

Although the above-outlined steps represent the preferred mode for practicing the present invention, it will be apparent to those skilled in the art that the above described approach can vary in accordance with techniques known in the art.

The following example is given to illustrate embodiments of the present invention as it is presently preferred to practice. It will be understood that this example is illustrative, and that the invention is not to be considered as restricted thereto except as indicated in the appended claims.

EXAMPLE

Cloning of BanI Restriction Endonuclease Gene

1. DNA purification:

10 g of frozen *Bacillus aneurinolyticus* (IAM 1077) cells were thawed on ice for 1 hour then resuspended in 20 ml of 25% sucrose, 50mM Tris pH 8.0. 10 ml of 0.25M EDTA pH 8.0, and 6 ml of 10 mg/ml lysozyme in 0.25M Tris pH 8.0 were added. The suspension was kept on ice for 2 hours, then lysed by the addition of 24 ml of 1% Triton X-100, 50mM Tris pH 8.0, 67mM EDTA and 5 ml of 10% SDS. The solution was extracted with 70 ml of phenol, (previously equilibrated with 0.5M Tris pH 8.0), and 60 ml of Chloroform. The emulsion was centrifuged at 10K rpm for 30 minutes to separate the phases. The viscous upper phase was transferred to a new bottle and extracted with phenol and chloroform once more. The emulsion was again centrifuged then the upper phase was dialyzed against four changes of DNA buffer (10mM Tris pH 8.0, 1mM EDTA). The dialyzed solution was then digested with RNase at a final concentration of 200 ug/ml for 1 hour at 37°C. The DNA was then precipitated by the addition of 5M NaCl to a final concentration of 0.4M, and 0.55 volumes of isopropyl alcohol. The precipitated DNA was spooled onto a glass rod, air-dried, then dissolved in DNA buffer to a concentration of approximately 450 ug/ml and stored at 4°C.

2. Digestion of DNA:

30 ug of *B.aneurinolyticus* DNA was diluted into 300 ul of HindIII restriction endonuclease digestion buffer (10mM Tris pH 7.5, 10mM MgCl₂, 10mM mercaptoethanol, 50mM NaCl). 30 units of HindIII restriction endonuclease were added and the solution was incubated at 37°C for 1 hr, then digestion was terminated by heating to 72°C for 12 minutes.

3. Ligation and transformation:

6 ug (60ul) of HindIII-digested *B.aneurinolyticus* DNA was mixed with 3 ug (15 ul) of HindIII-cleaved and dephosphorylated pBR322 (ATCC 37017). 20 ul of 10X ligation buffer (500mM Tris pH 7.5, 100mM MgCl₂, 100mM DTT, 5mM ATP), and 105 ul of sterile distilled water were added to bring the volume to 200 ul. 5 ul of T4 DNA ligase was added and the solution was incubated at 17°C for 3 hours. The solution was sterilized by extraction with 20 ul of chloroform, then clarified by microcentifugation for 15 sec. 85 ul of the ligation solution was mixed with 700 ul of SSC/CaCl₂ (50mM NaCl, 5mM Na₃Citrate, 67mM CaCl₂) and 1.4 ml of ice-cold, competent *E.coli* RR1 (ATCC 31343) cells were added. The solution was incubated at 44°C for 4 mins, then 12.5 ml of Luria-broth (L-broth) was added and incubation was continued at 37°C for 3 hr.

## 4. Cell Library:

The transformed culture was gently centrifuged, the supernatant was discarded and the cells were resuspended in 1.0 ml of L-broth. 200 ul portions of the resuspended cells were plated onto Luria-agar (L-agar) plates containing 100 ug/ml ampicillin. The plates were incubated overnight at 37°C. The transformed cells that grew up on the surfaces of the plates were collected together by flooding each of the plates with 2.5 ml of 10mM Tris pH 7.5, 10mM MgCl2, scraping the colonies together, and pooling the suspensions into a single tube.

## 5. Plasmid Library:

2.0 ml of the cell library was inoculated into 500 ml of L-broth containing 100 ug/ml ampicillin. The culture was shaken overnight at 37°C then centrifuged at 4K rpm for 5 minutes. The supernatant was discarded and the cell pellet was resuspended in 10 ml of 25% sucrose, 50mM Tris pH 8.0, at room temperature. 5 ml of 0.25M EDTA, pH 8.0, and 3 ml of 10 mg/ml lysozyme in 0.25M Tris pH 8.0 were added. The solution was kept on ice for 1 hour, then 12 ml of 1% Triton X-100, 50mM Tris pH 8.0, 67mM EDTA was added and the suspension was gently swirled to induce cell lysis.

The lysed mixture was transferred to a 50 ml tube and centrifuged for 45 min. at 17K rpm, 4°C. The supernatant was removed with a pipette. 20.0 gm of solid CsCl was weighed into a 50 ml plastic screw-cap tube and 22.0 gm of supernatant was pipetted into the tube and mixed. 1.0 ml of 5 mg/ml ethidium bromide in 10mM Tris pH 8.0, 100mM NaCl, 1mM EDTA was added. The solution was transferred to two 5/8 in. x 3 in. centrifuge tubes and spun in a Beckman Ti70 rotor for 42 hours at 44K rpm, 17°C. To collect the plasmids, the tubes were opened, illuminated with ultraviolet light, and the lower of the two fluorescent bands was collected by syringe. The lower band from each tube was combined and the ethidium bromide was removed by extracting four times with an equal volume of water-saturated, ice-cold N-Butanol.

The extracted solution was dialyzed against 4 changes of DNA buffer, then the nucleic acid was precipitated by the addition of 2 vols. of isopropanol and sufficient 5M NaCl to reach a final concentration of 0.4M. The solution was stored overnight at -20°C then centrifuged for 15 min.at 15K rpm, 0°C. The supernatant was discarded, the pellet was air-dried for 15 min. then dissolved in 500 ul of 10mM Tris pH 7.5, 1mM EDTA and stored at -20°C. The plasmid DNA concentration was found to be approximately 100 ug/ml.

## 6. Digestion of the Plasmid Library:

9 ug (90 ul) of the plasmid library was diluted into 300 ul of BanI restriction endonuclease digestion buffer (10mM Tris pH 7.5, 10mM MgCl2, 10mM mercaptoethanol, 10mM NaCl). 25 units (25 ul) of BanI restriction endonuclease were added and the tube was incubated at 37°C for 3 hr. The reaction was terminated by heating to 72°C for 12 minutes.

## 7. Transformation:

20 ul (0.6 ug) of the digested library was mixed with 130 ul of SSC/CaCl2 (section 3) and 300 ul of ice-cold, competent, E.coli RR1. The mixture was warmed to 42°C for 3 min. then briefly microfuged. The cell pellet was resuspended in 150 ul of L-broth and plated onto an L-agar containing 100 ug/ml ampicillin. The plate was incubated overnight at 37°C. BanI digestion reduced the number of transformants $10^3$-fold compared with transformation by undigested plasmids. Twenty eight colonies were picked from the survivors of the BanI digestion; each was inoculated into 10 ml of L-broth containing ampicillin, to prepare a miniculture, and streaked onto an L-agar plate containing ampicillin, to prepare a master stock.

## 8. Analysis of surviving individuals:

Twenty eight of the surviving colonies obtained from section 7 were grown into 10 ml cultures and the plasmids that they carried were prepared by the following miniprep purification procedure, adapted from the method of Birnboin and Doly, *Nucleic Acids Res.* 7: 1513 (1979).

## Miniprep Procedure:

Each culture was centrifuged at 8K rpm for 5 minutes; the supernatant was discarded and the cell pellet was resuspended in 1.0 ml of 25mM Tris, 10mM EDTA, 50mM glucose, pH 8.0, containing 1mg/ml lysozyme. After 10 minutes at room temperature, 2.0 ml of 0.2M NaOH, 1% SDS was added to each tube and the tubes were shaken to lyse the cells, then placed on ice. Once the solutions had cleared, 1.5 ml of 3M sodium acetate, pH 4.8, was added to each and shaken. The precipitates that formed were spun down at 15K rpm, 4°C for 10 minutes. Each supernatant was poured into a centrifuge tube containing 3 ml of isopropanol and mixed. After 10 minutes at room temperature, the tubes were spun at 15K rpm for 10 minutes to pellet the precipitated nucleic acids: The supernatants were discarded and the pellets were air-dried at room temperature for 30 minutes. Once dry, the pellets were resuspended in 850 ul of 10mM Tris, 1mM EDTA, pH 8.0. 75 ul of 5M NaCl was added to each and the solutions were transferred to Eppendorf tubes containing 575 ul of isopropanol, and again precipitated for 10 minutes at room temperature. The tubes were then spun for 45 seconds in a microfuge, the supernatants were discarded and the pellets were air-dried. The pellets were then dissolved in 500 ul of 10mM Tris, lmM EDTA, pH 8.0, containing 100 ug/ml RNase and incubated for 1 hour at 37°C to digest the RNA. The DNA was precipitated once more by the addition of 50 ul of 5M NaCl followed by 350 ul of isopropanol. After 10 minutes at room temperature, the DNA was spun down by centrifugation for 45 seconds, the supernatants were discarded and the pellets were redissolved in 150 ul of 10mM Tris 1mM EDTA, pH 8.0. The plasmid minipreps were subsequently analyzed by digestion with BanI and HindIII.

## 9. BanI Methylase Gene Clones:

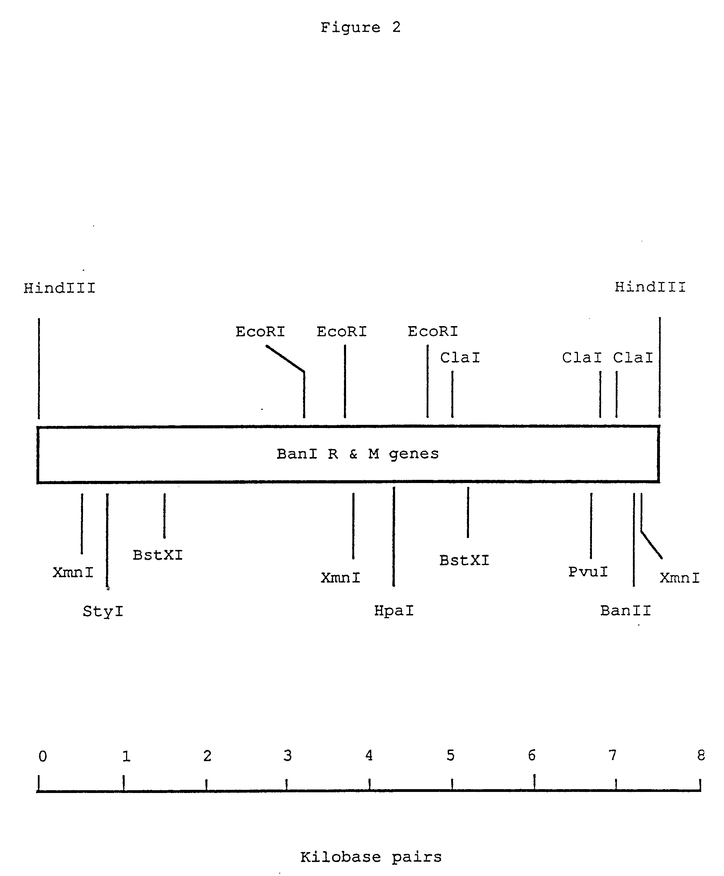

Approximately half of the 28 plasmids that were analyzed were found to be sensitive to BanI digestion and to carry diverse HindIII fragments of *B.aneurinolyticus* DNA. These plasmids were spurious and they were discarded. The remaining 12 plasmids were found to be resistant to BanI digestion and to carry a 7.5 Kb HindIII fragment in common. Several of the plasmids carried additional common fragments, presumably chromosomal neighbours of the 7.5 Kb fragment. The presence of the additional fragments did not affect the properties of the plasmids, so for simplicity only plasmids carrying the 7.5 Kb fragment were analyzed in detail (figure 2). These plasmids, typical of which is pGW118RM 2-1, were shown to encode not only the BanI modification methylase but also the BanI restriction endonuclease.

## 10. BanI Restriction Gene Clone:

pGW118RM 2-1, and similar plasmids, were found to encode and express the BanI restriction endonuclease by assaying extracts of *E.coli* RR1 that carried the plasmids.

Endonuclease Assay:

A 100 ml culture of the cells to be assayed was grown overnight at 37°C in L-broth containing 100 ug/ml ampicillin. The culture was centrifuged at 4K rpm for 5 min and the cell pellet was resuspended in 3.5 ml of 10mM KPO$_4$ pH 7.5, 10mM mercaptoethanol, 0.1mM EDTA. 0.5 ml of 10 mg/ml lysozyme in the same buffer was added and the suspension was left on ice for 2 hr. The suspension was frozen at -20°C, then thawed on ice. 1.0 ml of the thawed suspension was sonicated gently for three 10-second bursts to disrupt the cells. The sonicated extract was microcentrifuged for 5 min to remove cell debris and the supernatant was assayed for endonuclease activity in the following way:

60 ug (85 ul) of purified phage lambda DNA was diluted into 1200 ul of BanI restriction endonuclease digestion buffer (section 6). The solution was dispensed into 6 tubes, 150 ul into the first tube and 100 ul into each of the remaining 5 tubes. 7.5 ul of the extract was added to the first tube to achieve 1 ul extract/ug DNA. 50 ul was then removed from the first tube and transferred to the second tube to achieve 0.3 ul/ug. 50ul serial transfers were continued into tubes 3 (0.1 ul/ug), 4 (0.03 ul/ug) and 5 (0.001 ul/ug). the sixth tube received no extract and served as a negative control. The tubes were incubated at 37°C for one hour, then 20 ul from each was analyzed by gel electrophoresis. The extract was found to contain approximately 1x10$^4$ units of BanI restriction endonuclease per ml, which corresponds to about 1x10$^5$ units per gram of cells.

## 11. Phage-resistance of the BanI RM clones:

Some restriction-modification systems express the restriction phenotype when they are cloned into *E.coli*, and some do not. The restriction phenotype is the ability of cells to survive infection by phages and the inability of phages to reproduce. *E.coli* RR1 carrying pGW118RM 2-2, or other plasmids containing the 7.5 Kb HindIII fragment, displays a strong restriction phenotype. Lambdoid phages plaque with an efficiency of approximately 10$^{-6}$ on these clones compared to an efficiency of 1 on the same strain carrying pBR322.

## 12. Transfer of the 7.5 Kb fragment to pUC19:

5 ug (50 ul) of purified pGW118RM 2-1 DNA was prepared in 100 ul of HindIII restriction endonuclease digestion buffer (section 2). 40 units each of the restriction endonucleases HindIII, BamHI and PstI were added. The solution was incubated at 37°C for 1 hr then digestion was terminated by heating at 75°C for 15 min. HindIII was used to excise the 7.5 Kb fragment from the vector. BamHI and PstI, which cleave pBR322, but do not cleave the fragment, were used to digest the vector and prevent its re-emergence during the subsequent ligation.

3 ug (60 ul) of the digested pGW118RM 2-1 DNA was mixed with 1.5 ug (7.5 ul) of HindIII-cleaved and dephosphorylated pUC19. 10 ul of 10X ligation buffer (section 3) and 22.5 ul of sterile distilled water were added to bring the volume to 100 ul. 4 ul of T4 DNA ligase was added and the solution was incubated at 17°C for 4 hr. The ligation was sterilized by extraction with 10 ul of chloroform, then clarified by brief microcentrifugation. 22.5 ul of the sterile ligation was mixed with 100 ul CaCl$_2$/SSC (section 3) and 200 ul of competent, ice-cold, *E.coli* RR1. The mixture was incubated at 42°C for 5 min, then diluted into 10 ml of L-broth and incubated at 30°C overnight. 25 ul aliquots of the culture were then plated onto L-agar plates containing ampicillin and transformants were recovered after incubation at 30°C.

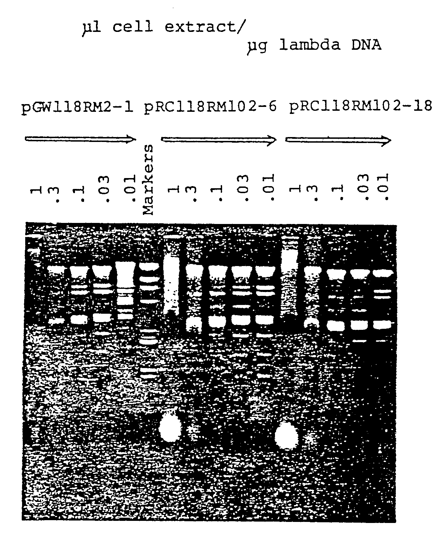

28 transformant colonies were picked and screened by the miniprep procedure (section 8) to identify plasmids composed of pUC19 with the 7.5 Kb fragment inserted at the HindIII site. Two plasmids were found to possess this structure, pRC118RM 102-6 and pRC118RM 102-18. They carry the 7.5 Kb fragment in opposite orientations and they exhibit complete resistance to BanI digestion. A crude cell extract of *E.coli* RR1 carrying each of these plasmids was assayed for BanI restriction endonuclease activity (figure 3). The extracts were found to contain substantially more BanI restriction endonuclease than the parental clone, in excess of 1x10$^5$ units/ml of extract (>1x10$^6$ units/gm cells).

*E.coli* RR1 carrying pRC118RM 102-6 or pRC118RM 102-18 is the preferred host from which the BanI restriction endonuclease can be purified. The strain should be grown to stationary phase at 37°C in a fermenter, in L-broth containing ampicillin. The cells should then be collected by centrifugation and either broken immediately for extract preparation, or stored frozen at -70°C until it is convenient to do so.

**Claims**

1. A cloning vector which includes a BanI restriction endonuclease gene.

2. A transformed host containing the cloning vector of claim 1.

3. The cloning vector of claim 1, wherein the BanI gene is excised from *Bacillus aneurinolyticus* IAM 1077.

4. A cloning vector which includes a BanI modification gene.

5. A transformed host containing the cloning vector of claim 4.

6. A method of cloning a BanI restriction endonuclease gene which comprises:

    (a) forming a library from DNA from *B.aneurinolyticus*

    (b) isolating clones which contain the BanI modification gene;

    (c) screening clones containing the modification gene; and

    (d) isolating clones which also contain the BanI restriction endonuclease gene.

7. The method of claim 6, wherein the library is formed by the steps of:

    (a) purifying DNA from *B.aneurinolyticus* IAM 1077;

    (b) digesting the purified DNA to form DNA fragments;

    (c) ligating the fragments into a cloning vector;

    (d) transforming a host cell with the cloning vector of step (c) to form a cell library; and

    (e) purifying recombinant vectors from the cell library to form a plasmid library.

8. The method of claim 7, wherein the cloning vector is pBR322.

9. The method of claim 7, wherein the host cell is a strain of *E.coli* which is mcrB⁻ and hsdR⁻.

10. The method of claim 7, wherein the clone containing the BanI modification gene is isolated by digesting the plasmid library with BanI to form a digestion pool, transforming the digestion pool into a host cell, and selecting clones containing the modification gene.

11. A method for producing BanI restriction endonuclease comprising:

    (a) purifying DNA from *B.aneurinolyticus*;

    (b) digesting the purified DNA with an appropriate restriction endonuclease to form DNA fragments;

    (c) ligating the fragments into the cloning vector to form a DNA mixture;

    (d) transforming a host cell with the DNA mixture of step (c) to form a library;

    (e) isolating clones which contain the BanI modification methylase gene;

    (f) screening clones containing the BanI modification methylase gene, and isolating clones which also contain the BanI restriction endonuclease gene;

    (g) culturing the host cells containing the clones of step (f); and

    (h) recovering BanI restriction endonuclease from the culture.

12. The method of claim 11, wherein the cloning vector is a plasmid or viral DNA molecule.

13. The method of claim 12, wherein the plasmid is pBR322.

14. The method of claim 12, wherein the plasmid is pUC19.

Figure 1

Figure 2

Kilobase pairs

µl cell extract/
µg lambda DNA

pGW118RM2-1 pRC118RM102-6 pRC118RM102-18